# EUROPEAN PATENT SPECIFICATION

(11) **EP 3 992 186 B1**
(45) Date of publication and mention of the grant of the patent: **22.05.2024**
(21) Application number: 20833005.0
(22) Date of filing: 04.06.2020
(51) Int. Cl.: C07D 301/12, C07D 303/16, C07D 303/04, C07D 303/40, C07D 493/04, C07D 493/10, C07D 303/27, C07D 303/44, C07D 493/08, C07D 493/20

(54) **SAFE, ENVIRONMENTALLY FRIENDLY AND CONTROLLABLE PROCESS FOR SYNTHESIZING DIEPOXIDE**
SICHERES UMWELTFREUNDLICHES UND STEUERBARES VERFAHREN ZUR SYNTHESE VON DIEPOXID
PROCÉDÉ SÛR, RESPECTUEUX DE L'ENVIRONNEMENT ET MAÎTRISABLE DE SYNTHÈSE DE DIÉPOXYDE

(30) Priority: 27.06.2019 CN 201910564540
(43) Date of publication of application: 04.05.2022
(73) Proprietor: Jiangsu Tetra New Material Technology Co., Ltd., Taizhou, Jiangsu 225300 (CN)
(72) Inventor: HAN, JianWei, taizhou, Jiangsu 225300 (CN); CHANG, YangJun, taizhou, Jiangsu 225300 (CN); YANG, Sheng, taizhou, Jiangsu 225300 (CN); JIA, Quan, taizhou, Jiangsu 225300 (CN); CAO, XiangMing, taizhou, Jiangsu 225300 (CN)
(74) Representative: Botti & Ferrari S.p.A.
(86) International application number: PCT/CN2020/094348
(87) International publication number: WO 2020/259245

(56) References cited:
- WO-A1-2019/102102
- CN-A- 105 491 839
- CN-A- 107 216 613
- CN-A- 108 003 328
- CN-A- 108 101 868
- CN-A- 109 096 228
- CN-A- 109 912 544
- CN-A- 110 183 401
- YOKOTA, Kozo et al.: "Occupational Dermatitis from a One-Component Naphthalene Type Epoxy Adhesive", Industrial Health, vol. 40, no. 1,, 31 December 2002 (2002-12-31), XP055773241, ISSN: 0019-8366, DOI: 20200706170549X
- QIU, Junfeng et al.: "Study on the synthesis of dicyclopentadiene dioxide", Thermosetting Resin, vol. 30, no. 4, 31 July 2015 (2015-07-31), pages 5-8, XP055886875, ISSN: 1002-7432
- FENG, Shaojing et al.: "Preparation of 3,4-epoxycyclohexylcarboxylic acid-3 ,4 -epoxycyclohexylmethyl Ester", Hecheng Shuzhi ji Suliao - China Synthetic Resin and Plastics, vol. 35, no. 6, 31 December 2018 (2018-12-31), pages 10-14, XP009533520, ISSN: 1002-1396

## Description

### Field of application

The present disclosure relates to the field of epoxide synthesis, and particularly to a safe, environmentally friendly and controllable method for preparing cycloaliphatic diepoxides.

### Prior Art

Epoxy resin and cured products thereof have been widely used in aerospace, microelectronics packaging, motor insulation and other important industrial fields due to their excellent machinability, thermal stability, electrical insulation and UV radiation resistance, etc. With the increasing performance and functional requirements of polymer materials in modern industry, the researches on the synthesis and performance of epoxy resins have been very active in recent years.

Epoxy resins are generally liquid with a low viscosity at room temperature before curing, they can often be used directly for construction operations of coatings and electronic packaging materials without solvent dilution, which is convenient for potting, pouring or vacuum injection. They have been widely used in the fields of VLSI packaging, printed circuit board manufacturing, special light-curing coatings, and high-capacity and high-temperature-resistant motor insulation materials for vacuum pressure impregnation technology due to excellent comprehensive properties in recent years.

Since methods for preparing epoxy resins in prior art mostly use traditional catalysts, such catalysts usually contain heavy metal ions (such as tungsten W), so the product also contains a certain amount of heavy metal ion residues; due to the influence of heavy metal ion-containing catalysts, the final product also has heavy metal ion residues, which also affects the curing speed (i.e. gel time) of the product, thereby affecting the cross-linking in the molecule during curing, prolonging the gel time of the product, and affecting the production efficiency and product quality.

CN109096228A discloses a method for preparing dipentene dioxide, comprising the following steps: (1) mixing limonene, toluene, acetic anhydride and sodium acetate to obtain a mixture, and cooling the mixture to no more than 15 °C; (2) adding hydrogen peroxide to the mixture obtained in step (1) to react; (3) adjusting a resulting material obtained in step (2) to an alkaline pH value, standing for stratification, and retaining a first upper organic phase; (4) adding an aqueous sodium sulfite solution to the first organic phase obtained in step (3), standing for stratification to obtain a second upper organic phase, removing solvent from the second organic phase to obtain a crude product; and (5) purifying the crude product obtained in step (4) to obtain dipentene dioxide.

### Summary of the invention

In view of some problems existing in the prior art, a first aspect of the present invention provides a method for preparing cycloaliphatic diepoxides comprising the steps of:
- mixing a diene compound, a carboxylic acid anhydride, an alkaline salt, and a solvent, and cooling;
- dropwise adding a hydrogen peroxide solution thereto over 1-12 h;
- standing for layering to obtain an underlayer of an organic phase I, washing the organic phase I with a washing liquid, and standing for layering to obtain an underlayer of an organic phase II; and
- purifying the organic phase II.

In some embodiments, a molar ratio of the hydrogen peroxide to the diene compound is (1-5.5): 1.

In some embodiments, a molar ratio of the carboxylic acid anhydride to the diene compound is (1-3.5): 1.

In some embodiments, a weight ratio of the washing liquid to the solvent is 1: (2.5-3.5).

In some embodiments, the washing liquid includes an inorganic alkali solution and a reducing salt.

In some embodiments, the reducing salt accounts for 0.5-5 wt% of the washing liquid.

In some embodiments, the purifying includes a vacuum distillation and a two-stage thin film distillation.

In some embodiments, a temperature of a first-stage thin film distillation is 40-60°C, and a temperature of a second-stage thin film distillation is 80-100°C.

A second aspect of the present invention provides a cycloaliphatic diepoxide prepared by the method described in above technical embodiments.

In some embodiments, the cycloaliphatic diepoxide is one or more selected from the group consisting of 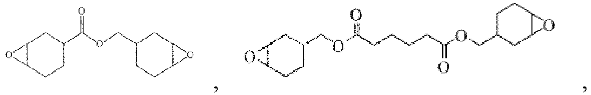 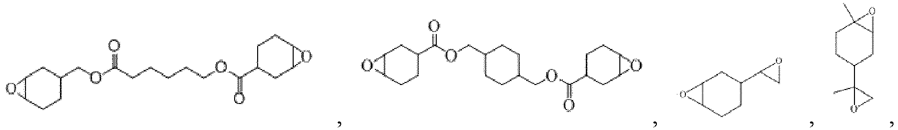 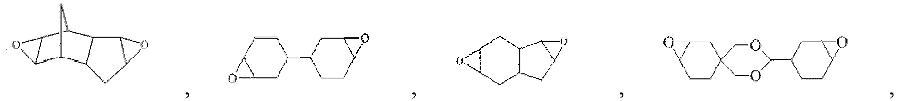 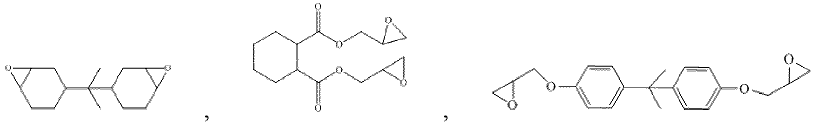 and

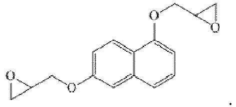

Compared with the prior art, the present disclosure has the following beneficial effects:
The reaction system of the present disclosure is simple, environmentally friendly, safe and controllable, and the production cost is low, which can meet the technical and economic requirements. The obtained cycloaliphatic diepoxides have high purity, high yield, low solvent content, low chroma and low halogen content, which are suitable for large-scale industrial production.

### Detailed description of the embodiments

The present disclosure is hereunder illustrated with specific embodiments, but is not limited to the specific examples given below.

The first aspect of the present invention provides a method for preparing cycloaliphatic diepoxides comprising the steps of:
- mixing a diene compound, a carboxylic acid anhydride, an alkaline salt, and a solvent, and cooling;
- dropwise adding a hydrogen peroxide solution thereto over 1-12 h;
- standing for layering to obtain an underlayer of an organic phase I, washing the organic phase I with a washing liquid, and standing for layering to obtain an underlayer of an organic phase II; and
- purifying the organic phase II.

In one embodiment, the method for preparing cycloaliphatic diepoxides at least comprises the steps of:
(1) mixing a diene compound, a carboxylic acid anhydride, an alkaline salt, and a solvent, and cooling;
(2) dropwise adding a hydrogen peroxide solution thereto over 1-12 h;
(3) standing for layering to obtain an underlayer of an organic phase I, washing the organic phase I with a washing liquid, and standing for layering to obtain an underlayer of an organic phase II; and
(4) purifying the organic phase II.

### Step (1),

In one embodiment, a cooling temperature is 5-20°C, preferably 10-15°C, and more preferably 13°C.

If the temperature is too low, an initial reaction rate is slow, which is likely to generate the acid accumulation to cause difficulties in controlling the temperature in later stage; if the temperature is too high, it is easy to cause the decomposition of hydrogen peroxide, resulting in an incomplete reaction. The reaction temperature is easy to control and the side reactions are few by cooling the temperature to the above range and dropwise adding hydrogen peroxide; the reaction can be carried out smoothly and gently in a low temperature due to the strong oxidizing property of hydrogen peroxide and exothermic reaction, which can prevent excessive heat release and rapid temperature rise in the early stage of the reaction to cause out of control and production safety risks.

In one embodiment, the diene compound is one or more selected from the group consisting of 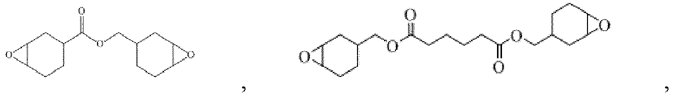 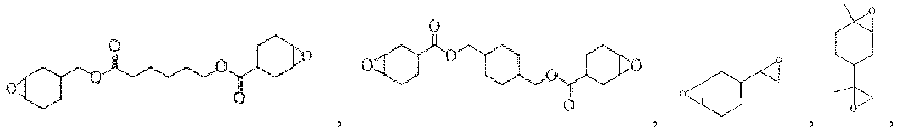 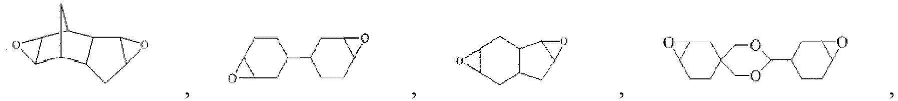 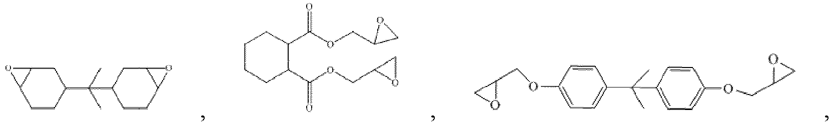 and 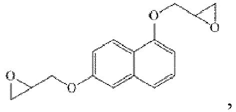 and there is no restriction on the manufacturer of the diene compound.

In one embodiment, the molar ratio of the carboxylic acid anhydride to the diene compound is (1-3.5): 1.

The number of carbon atoms of the carboxylic acid anhydride is preferably 3-7; and the carboxylic acid anhydride is preferably one or more selected from a group consisting of acetic anhydride, propionic anhydride, succinic anhydride, and maleic anhydride.

The carboxylic acid anhydride is preferably acetic anhydride, a purity of the acetic anhydride is preferably 98-100 wt%, and there is no restriction on the manufacturer of the acetic anhydride.

Examples of the alkaline salt include sodium carbonate, sodium hydroxide, potassium carbonate, potassium hydroxide, and sodium acetate; the alkaline salt is preferably sodium acetate, and there is no restriction on the manufacturer of the sodium acetate.

In the present disclosure, the pH value of the reaction system is regulated by the acidic substances produced in the neutralization reaction of the alkaline salt, which can avoid the out of control of the temperature caused by the acid enrichment in the system, avoid the decomposition of the obtained epoxide, and promote the progress of the oxidation reaction.

In one embodiment, the solvent is at least one of an aromatic solvent, a chlorinated solvent, and an ester solvent.

Examples of the aromatic solvent include benzene, toluene, ethylbenzene, xylene, trimethylbenzene etc., examples of the chlorinated solvent include chloroform, dichloroethane, chlorobenzene, 1,3-dichloropropane, 1,2-dichloroethane, etc.; examples of the ester solvent include ethyl acetate, propyl acetate, butyl acetate, ethyl propionate, dimethyl carbonate, diethyl carbonate, etc.

In one embodiment, examples of the solvent include methyl isobutyl ketone, methanol, ethanol, tert-butanol, etc.

The solvent is preferably chloroform, and there is no restriction on the manufacturer of the chloroform.

In one embodiment, the weight ratio of the diene compound to the solvent is 1:(2-4.5), and preferably 1:3.

### Step (2),

In one embodiment, the process of step (2) is as follows: dropwise adding the hydrogen peroxide solution over 1-12 h, and then continuing the reaction for another 3-8 h.

The time for dropwise adding the hydrogen peroxide solution is preferably 3-6 h, and more preferably 4.5 h.

The reaction is preferably continued for another 3-5 h, and more preferably 4 h.

In one embodiment, the temperature of the system in step (2) is 20-40°C, and preferably 30°C.

In one embodiment, the pH value of the system in step (2) is 3.0-4.5, and preferably 3.7.

During the preparation process, the applicant accidentally found that the reaction efficiency can be effectively improved by slowly adding hydrogen peroxide dropwise within 1-8 h and continuing the reaction at 20-40°C after the completion of dropwise addition, i.e. in a short reaction time, the obtained epoxy resin has high yield, high purity, and the color is colorless and transparent, which may be attributed to the fact that the reaction can be carried out safely, smoothly and efficiently by controlling the system temperature and addition time within the above range; If the temperature is high and the time is short, the reaction will be violent and uncontrollable, and the contact between materials will not be sufficient, which will not only cause the residue and self-polymerization of some diene substances, but also make the produced epoxy resins fully contact with oxidizing substances, promote oxidation, and reduce the purity and yield of the product; If the time is too long, the nucleophilic substances in the system are easy to react with the epoxy resin with large internal tension due to the lack of oxidizing substances in the acid system, thereby promoting the decomposition of the obtained epoxy resin and the generation of by-products, and reducing the yield.

In one embodiment, the concentration of the hydrogen peroxide solution is 30-70 wt%, preferably 35 wt%, and there is no restriction on the manufacturer of the hydrogen peroxide.

The molar ratio of the hydrogen peroxide to the diene compound is preferably (1-5.5): 1.

It has been experimentally found that the specific contents of hydrogen peroxide, carboxylic acid anhydride and diene compound are beneficial to improve the purity, yield and colorless transparency of the obtained aliphatic epoxy resin, which may be because when there is too much diene compounds, they will undergo self-polymerization reaction, thereby reducing the yield of the obtained product; when there is too much hydrogen peroxide in the system, some water will be produced, which will reduce the purity of the system, and also promote the hydrolysis of part of the obtained epoxides, as well as the oxidation of part of the diene compounds or the obtained epoxides into ketones, then various reactions such as decomposition and oxidation in the reaction system are carried out at the same time, which will reduce the yield and purity, and make the obtained resin turn yellow, thereby affecting its application in display lamp electronic products; when there is too much carboxylic acid anhydride, acid enrichment in the reaction system may be formed, which will promote the decomposition of the obtained epoxy resin, reduce the yield and purity, and also make the obtained resin turn light yellow or yellowish-brown.

### Step (3),

In one embodiment, the process of step (3) is as follows: standing for layering to obtain an underlayer of an organic phase I, adding a washing liquid thereto, and stirring to wash the organic phase I while maintaining a certain pH value; and standing for layering to obtain an underlayer of an organic phase II.

The pH value is preferably 10-12, and more preferably 11.

The stirring time is preferably 20-40 min, and more preferably 30 min.

In one embodiment, the weight ratio of the washing liquid to the solvent is 1: (2.5-3.5), preferably 1:3.

The washing liquid preferably includes an inorganic alkali solution and a reducing salt.

The concentration of the inorganic alkali solution is preferably 10-50 wt%, more preferably 30 wt%.

The reducing salt preferably accounts for 0.5-5 wt% of the washing liquid, more preferably 1 wt%.

Examples of the inorganic alkali include sodium hydroxide, potassium hydroxide, sodium carbonate, potassium carbonate, sodium bicarbonate, potassium bicarbonate, ammonium hydroxide, etc.; the inorganic alkali is preferably sodium hydroxide, and there is no restriction on the manufacturer of the sodium hydroxide.

Examples of the reducing salt include sodium sulfite, ferrous chloride, sodium thiosulfate, ferrous sulfate, etc.; the reducing salt is preferably sodium sulfite, and there is no restriction on the manufacturer of the sodium sulfite.

It has been experimentally found that washing the obtained reaction solution with an inorganic alkali and a reducing salt at the same time is beneficial to the improvement of the yield and purity of the final product, as well as the formation of colorless and transparent epoxy resin, which may be because the interaction between the reducing salts and the oxidizing substances in the system during stirring can avoid further oxidation of the epoxy resin obtained in the system, thereby improving the purity; In addition, inorganic alkali can neutralize the residual acidic solution in the reaction solution to make it in a free ionic state in the system and transfer from the organic phase to the aqueous phase, and it can also continuously reduce the products of reducing substances and oxidizing substances during the neutralization process, thereby promoting the effect of oxidizing substances and improving the purity of the system.

The applicant also found that adjusting the content of the reducing salts in the washing liquid can further optimize the yield, purity and chroma of the products, possibly because when there is too much reducing salts, the neutralization of the alkali solution in the system is saturated, and the reducing salts are easily oxidized to produce acid enrichment, so that some organic acids remain in the organic phase, thereby promoting the decomposition of epoxy resins.

In addition, it was unexpectedly found that adjusting the ratio of the washing liquid to the organic solvent can further optimize the yield, purity and chroma of the products, possibly because the small-molecular impurities in the organic phase cannot be completely neutralized and reduced when the content of washing liquid is small, or the small-molecular impurities remain in the organic phase as the saturation is reached; However, when the content of the washing liquid is large, the organic phase tends to shrink inward to form microspheres during stirring due to the large steric hindrance and the hydrophobic ring structure of the epoxy resin, which will affect the free movement of small molecule impurities into the aqueous phase, thereby reducing the purity and chroma of the products.

In one embodiment, the process of step (3) is as follows: standing for layering to obtain an underlayer of an organic phase I, adding a washing liquid thereto, and stirring to wash the organic phase I while maintaining at a certain pH value; standing for layering to obtain an underlayer of an organic phase II; and then washing with an aqueous solution to obtain an underlayer of an organic phase 3.

The weight ratio of the aqueous solution to the solvent is preferably 1:(2.5-3.5), more preferably 1:3.

The aqueous solution preferably contains small-molecular amines and tannins; and the weight ratio of the small-molecular amines to tannins is preferably (3-5): 1, more preferably 4: 1.

The small-molecular amines are preferably any one of monoethanolamine, diethanolamine, hydroxyethylethylenediamine, and N-acetylethylenediamine; and more preferably N-acetylethylenediamine.

It has been experimentally found that washing the product with tannins and small-molecular amines can further optimize the yield, purity, and chroma of the products, possibly because tannin is a compound with a large molecular weight and is soluble in water, it can interact with the aliphatic segments in the epoxy resin during washing and stirring, so that the relatively concentrated molecular chains in the organic phase are stretched, the space occupied volume is increased, and the relative freedom of molecules of the small-molecular impurities in the system is improved. On the other hand, the force between tannins and small molecule impurities is higher than the force between small molecules and epoxy resins in the process of stirring, so the washing efficiency is improved under the dual action; but the tannin has a large molecular weight, a low free rotation of chain segments, and a low contact ability with small molecules in the system, while the small-molecule amines are more likely to move between the interface of the aqueous phase and the organic phase under the action of tannins, thereby bringing out small molecules to interact with tannins and acting as a bridge; In addition, small-molecule amines can interact with trace metal elements in the system to reduce impurities, improve purity, and optimize chroma; at the same time, in the presence of nucleophilic groups, small-molecular amines can form N⁺ and further interact with nucleophilic groups, thereby weakening the interaction between nucleophilic groups and epoxy resins, reducing the decomposition of epoxy, and reducing impurities.

It has been experimentally found that adjusting the ratio of tannins to small-molecular amines can further optimize the yield, purity and chroma. It may be because when the content of tannins is small, the organic phase that shrinks inward as microspheres during stirring has a low degree of extension, and the molecular chain with large steric hindrance forms a diaphragm, which hinders the free movement of small-molecular impurities and small-molecular amines, thereby reducing the removal rate of impurities; when the content of small-molecular amines is small, the bridge effect is low, and the interaction between small-molecular impurities and the aqueous phase is weak, which causes some impurities to remain in the organic phase, thereby reducing the purity and chroma of the product.

### Step (4),

In one embodiment, the process of step (4) is as follows: first purifying by a vacuum distillation, and then purifying by a two-stage thin film distillation.

In one embodiment, the vacuum degree of the vacuum distillation is -0.05--0.1 MPa, the temperature is 25-60°C, and the time is 0.5-1 h; preferably, the vacuum degree is -0.07 MPa, the temperature is 42°C, and the time is 0.75 h.

In one embodiment, the temperature of a first-stage thin film distillation is 40-60°C, and the temperature of a second-stage thin film distillation is 80-100°C; preferably, the temperature of the first-stage thin film distillation is 50°C, and the temperature of the second-stage thin film distillation is 90°C.

The pressure of the first-stage thin film distillation in the two-stage thin film distillation is preferably 200-250 Pa, and more preferably 220 Pa.

The pressure of the second-stage thin film distillation in the two-stage thin film distillation is preferably 20-40 Pa, and more preferably 30 Pa.

The time of the first-stage thin film distillation in the two-stage thin film distillation is preferably 0.5-1.5 h, and more preferably 1 h.

The time of the second-stage thin film distillation in the two-stage thin film distillation is preferably 0.5-1.5 h, and more preferably 1 h.

There is no restriction on the material of the thin film. In the present disclosure, the material of the thin film is a scraper, and the material is stainless steel 316L.

The applicant discovered unexpectedly in the process of product purification that by using a two-stage thin film distillation and controlling the temperature and pressure of the two-stage distillations, the solvent content in the aliphatic epoxy resin can be effectively reduced to less than 0.1 %, the purification efficiency of aliphatic epoxy resin can be improved, i.e., the purification can be completed within 2 h, and the obtained aliphatic epoxy resin is colorless and transparent. This may be because using a low-temperature high-pressure thin film distillation, followed by a high-temperature low-pressure thin film distillation can effectively reduce the content of solvents, acid substances and other impurities of epoxy resin, and can effectively avoid the occurrence of side reactions during the distillation process, i.e., first using a low-temperature high-pressure thin film distillation can reduce the content of nucleophilic groups with a boiling point lower than the epoxy resin, which can avoid the decomposition of epoxy resins with large internal tension; in the initial distillation stage, the internal friction between the molecular chains is small, the viscosity is low, and the thermal parameters are also low, but the small molecules can move to the surface of the system relatively easily, which can be removed from the system in a short time; however, because the obtained epoxy resin has a certain steric hindrance, the free movement of small molecules is limited, and the solvent content in the obtained epoxy resin is still 1-5 %; the solvent in the system can be further reduced after a high-temperature low-pressure thin film distillation, it may be because the molecular chains are entangled together after the previous treatment, the internal friction force in the system is increased, the viscosity is increased, and the thermal parameters are also increased. The free movement of the molecular chains is increased under high temperature and low pressure, so that the internal solvent is further freed to the surface of the system, further removing the participating solvents or small-molecular impurities in the system, and improving the purity and colorless transparency of the epoxy resin.

However, distilling by a one-stage thin film distillation at a higher temperature for 10-20 h, the obtained epoxy resin is pale yellow having a solvent residue of 0.5-1 %. This is because the removal of solvent or small molecule impurities on the surface of the system is rapid under high temperature, and the degree of entanglement of molecular chains on the surface of the epoxy resin is high, which hinders the speed and total amount of solvents or other small-molecule impurities in the system to the surface. In addition, side reactions such as decomposition and self-polymerization of the non-uniform epoxy resin will occur under high temperature, which increases the instability of the system, thereby reducing the purity, increasing the solvent content and the degree of color development of the obtained epoxy resin.

The second aspect of the present invention provides a cycloaliphatic diepoxide prepared by the method described in above technical embodiments.

The cycloaliphatic diepoxide is preferably selected from the group consisting of 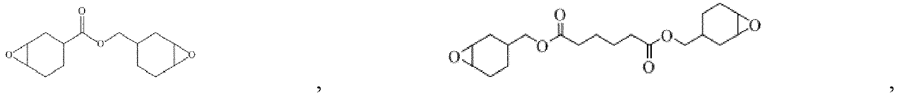 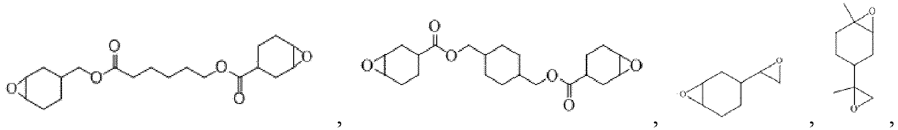 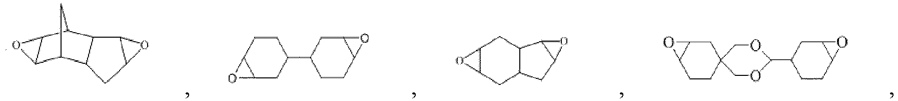 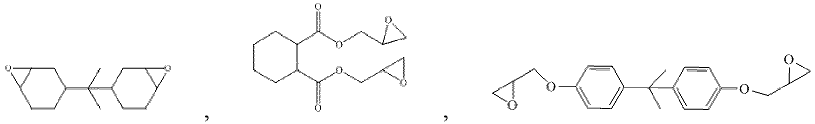 and

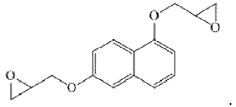

### Examples

Hereinafter, the present disclosure will now be described in further detail by way of examples. It is to be understood that the specific embodiments described herein are merely illustrative of the present application and are not intended to limit the present application. Unless otherwise stated, the raw materials used in the following examples are all commercially available.

### Example 1

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 30°C and a pH of 3.7, a hydrogen peroxide solution was added thereto dropwise over 5 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

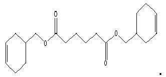

The molar ratio of the carboxylic acid to the diene compound was 2.4:1.

The carboxylic acid was acetic anhydride, and the purity of the acetic anhydride was 98%; the alkali salt was sodium acetate; and the solvent was chloroform.

The weight ratio of the diene compound to the solvent was 1:3.

The concentration of the hydrogen peroxide was 35%; and the molar ratio of the hydrogen peroxide to the diene compound was 3.6:1.

The weight ratio of the washing liquid to the solvent was 1:3.

The washing liquid was a mixture of an inorganic alkali solution and a reducing salt.

The inorganic alkali solution was a sodium hydroxide solution; the concentration of the inorganic alkali solution was 30 wt%.

The reducing salt was sodium sulfite; the content of the reducing salt was 1 wt% of the washing liquid.

A cycloaliphatic diepoxide was prepared by the above method.

The cycloaliphatic diepoxide was

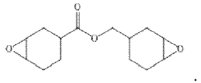

### Example 2

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 4.5 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

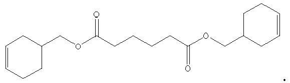

The molar ratio of the carboxylic acid to the diene compound was 2.35: 1.

The carboxylic acid was acetic anhydride, and the purity of the acetic anhydride was 98%; the alkali salt was sodium acetate; and the solvent was chloroform.

The weight ratio of the diene compound to the solvent was 1:3.

The concentration of the hydrogen peroxide was 35%; the molar ratio of the hydrogen peroxide to the diene compound was 3.6:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of the washing liquid.

A cycloaliphatic diepoxide was prepared by the above method.

The cycloaliphatic diepoxide was

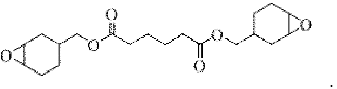

### Example 3

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 4.5 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

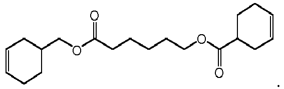

The molar ratio of the carboxylic acid to the diene compound was 2:1.

The carboxylic acid was acetic anhydride, and the purity of the acetic anhydride was 98%; and the alkali salt was sodium acetate; and the solvent was chloroform.

The weight ratio of the diene compound to the solvent was 1:3.

The concentration of the hydrogen peroxide was 35%; and the molar ratio of the hydrogen peroxide to the diene compound was 2.1:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of the washing liquid.

A cycloaliphatic diepoxide was prepared by the above method.

The cycloaliphatic diepoxide was

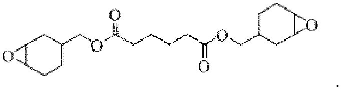

### Example 4

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 5 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

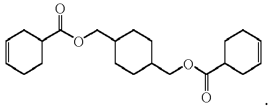

The molar ratio of the carboxylic acid to the diene compound was 3.5:1.

The carboxylic acid was acetic anhydride, and the purity of the acetic anhydride was 98%; and the alkali salt was sodium acetate; and the solvent was chloroform.

The weight ratio of the diene compound to the solvent was 1:3.

The concentration of the hydrogen peroxide was 35%; and the molar ratio of the hydrogen peroxide to the diene compound was 5: 1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of the washing liquid.

A cycloaliphatic diepoxide was prepared by the above method.

The cycloaliphatic diepoxide was

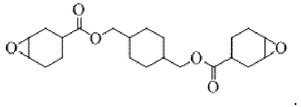

### Example 5

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by the vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

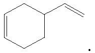

The molar ratio of the carboxylic acid to the diene compound was 2:1.

The carboxylic acid was acetic anhydride, and the purity of the acetic anhydride was 98%; and the alkali salt was sodium acetate; and the solvent was chloroform.

The weight ratio of the diene compound to the solvent was 1:2.5.

The concentration of the hydrogen peroxide was 35%; the molar ratio of the hydrogen peroxide to the diene compound was 3: 1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and a content of the reducing salt was 1 wt% of the washing liquid.

A cycloaliphatic diepoxide was prepared by the above method.

The cycloaliphatic diepoxide was

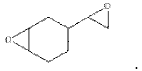

### Example 6

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

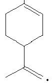

The molar ratio of the carboxylic acid to the diene compound was 2.3:1.

The carboxylic acid was acetic anhydride, and the purity of the acetic anhydride was 98%; the alkali salt was sodium acetate; and the solvent was chloroform.

The weight ratio of the diene compound to the solvent was 1:2.5.

The concentration of the hydrogen peroxide was 35%; and the molar ratio of the hydrogen peroxide to the diene compound was 3:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of the washing liquid.

A cycloaliphatic diepoxide was prepared by the above method.

The cycloaliphatic diepoxide was

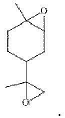

### Example 7

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

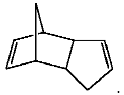

The molar ratio of the carboxylic acid to the diene compound was 2.4:1.

The carboxylic acid was acetic anhydride, and the purity of the acetic anhydride was 98%; and the alkali salt was sodium acetate; and the solvent was chloroform.

The weight ratio of the diene compound to the solvent was 1:3.

The concentration of the hydrogen peroxide was 35%; the molar ratio of the hydrogen peroxide to the diene compound was 3.5:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of washing liquid.

A cycloaliphatic diepoxide was prepared by the above method.

The cycloaliphatic diepoxide was

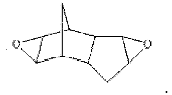

### Example 8

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

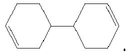

The molar ratio of the carboxylic acid to the diene compound was 2: 1.

The carboxylic acid was acetic anhydride, and the purity of the acetic anhydride was 98%; and the alkali salt was sodium acetate; and the solvent was chloroform.

The weight ratio of the diene compound to the solvent was 1:3.

The concentration of the hydrogen peroxide was 35%; the molar ratio of the hydrogen peroxide to the diene compound was 2.5:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and the content of the reducing salt was 1 wt% of the washing liquid.

A cycloaliphatic diepoxide was prepared by the above method.

The cycloaliphatic diepoxide was

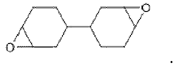

### Example 9

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added dropwise for 11 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The lower organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

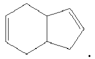

The molar ratio of the carboxylic acid to the diene compound was 2.5:1.

The carboxylic acid was acetic anhydride, and the purity of the acetic anhydride was 98%; and the alkali salt was sodium acetate; and the solvent was chloroform.

The weight ratio of the diene compound to the solvent was 1:2.5.

The concentration of the hydrogen peroxide was 35%; and the molar ratio of the hydrogen peroxide to the diene compound was 3.7:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and a content of the reducing salt was 1 wt% of the washing liquid.

A cycloaliphatic diepoxide was prepared by the above method.

The cycloaliphatic diepoxide was

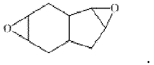

### Example 10

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

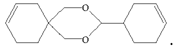

The molar ratio of the carboxylic acid to the diene compound was 2.2:1.

The carboxylic acid was acetic anhydride, and the purity of the acetic anhydride was 98%; the alkali salt was sodium acetate; and the solvent was chloroform.

The weight ratio of the diene compound to the solvent was 1:3.

The concentration of the hydrogen peroxide was 35%; and the molar ratio of the hydrogen peroxide to the diene compound was 3.3:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and a content of the reducing salt was 1 wt% of washing liquid.

A cycloaliphatic diepoxide was prepared by the above method.

The cycloaliphatic diepoxide was

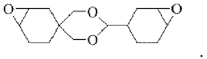

### Example 11

In this example, a method for preparing a cycloaliphatic diepoxide is provided, which comprises the following steps:
(1) A diene compound, a carboxylic acid, an alkaline salt, and a solvent were mixed, and cooled to 13°C;
(2) At a temperature of 20°C and a pH of 3.7, a hydrogen peroxide solution was added thereto dropwise for 8 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a two-stage thin film distillation, the temperature of the first-stage thin film distillation was 50°C, the distillation pressure was 220 Pa, the distillation time was 1 h, the temperature of the second-stage thin film distillation was 90°C, the distillation pressure was 30 Pa, and the distillation time was 1 h.

The diene compound was

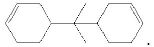

The molar ratio of the carboxylic acid to the diene compound was 2.2:1.

The carboxylic acid was acetic anhydride, and the purity of the acetic anhydride was 98%; and the alkali salt was sodium acetate; and the solvent was chloroform.

The weight ratio of the diene compound to the solvent was 1:3.

The concentration of the hydrogen peroxide was 35%; and the molar ratio of the hydrogen peroxide to the diene compound was 3.3:1.

The weight ratio of the washing liquid to the solvent was 1:3.

The washing liquid was a mixture of an inorganic alkali solution and a reducing salt.

The inorganic alkali solution was a sodium hydroxide solution; and the concentration of the inorganic alkali solution was 30 wt%.

The reducing salt was sodium sulfite; and a content of the reducing salt was 1 wt% of the washing liquid.

A cycloaliphatic diepoxide was prepared by the above method.

The cycloaliphatic diepoxide was

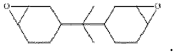

### Example 12

In this example, a method for preparing a cycloaliphatic diepoxide is provided, and its specific implementation is the same as that of Example 1, except that after obtaining the organic phase II in step (3), it is washed with an aqueous solution to obtain an underlayer of an organic phase 3; the weight ratio of the aqueous solution to the solvent is 1:3; the aqueous solution contains a small-molecular amine and a tannin; the weight ratio of the small-molecular amine and the tannin is 4:1; and the small-molecular amine is N-acetylethylenediamine.

### Example 13

In this example, a method for preparing a cycloaliphatic diepoxide is provided, and its specific implementation is the same as that of Example 12, except that the weight ratio of the aqueous solution to the solvent is 1:2.5; and the weight ratio of the small-molecular amine to the tannin is 3:1.

### Example 14

In this example, a method for preparing a cycloaliphatic diepoxide is provided, and its specific implementation is the same as that of Example 12, except that the weight ratio of the aqueous solution to the solvent is 1:3.5; and the weight ratio of the small-molecular amine to the tannin is 5:1.

### Example 15

In this example, a method for preparing a cycloaliphatic diepoxide is provided, and its specific implementation is the same as that of Example 12, except that the small-molecular amine is 3-methylenecyclobutylamine.

### Example 16

In this example, a method for preparing a cycloaliphatic diepoxide is provided, and its specific implementation is the same as that of Example 12, except that the weight ratio of the small-molecular amine to the tannin is 1: 1.

### Example 17

In this example, a method for preparing a cycloaliphatic diepoxide is provided, and its specific implementation is the same as that of Example 12, except that the weight ratio of the small-molecular amine to the tannin is 10:1.

### Example 18

In this example, a method for preparing a cycloaliphatic diepoxide is provided, and its specific implementation is the same as that of Example 1, except that the weight ratio of the washing liquid to the solvent is 1:1.

### Example 19

In this example, a method for preparing a cycloaliphatic diepoxide is provided, and its specific implementation is the same as that of Example 1, except that the reducing salt accounts for 7 wt% of the washing liquid.

### Example 20

In this example, a method for preparing a cycloaliphatic diepoxide is provided, and its specific implementation is the same as that of Example 1, except that the time for the dropwise addition of the hydrogen peroxide solution is 10 min, but based on safety considerations, it does not have operability.

### Example 21

In this example, a method for preparing a cycloaliphatic diepoxide is provided, and its specific implementation is the same as that of Example 1, except that the time for the dropwise addition of the hydrogen peroxide solution is 13 h.

### Example 22

In this example, a method for preparing a cycloaliphatic diepoxide is provided, and its specific implementation is the same as that of Example 1, except that the molar ratio of the carboxylic acid to the diene compound is 5: 1.

### Example 23

In this example, a method for preparing a cycloaliphatic diepoxide is provided, and its specific implementation is the same as that of Example 1, except that the molar ratio of the carboxylic acid to the diene compound is 1:1.

### Example 24

In this example, a method for preparing a cycloaliphatic diepoxide is provided, and its specific implementation is the same as that of Example 1, except that the molar ratio of the hydrogen dioxide to the diene compound is 7: 1.

### Example 25

In this example, a method for preparing a cycloaliphatic diepoxide is provided, which comprises the following steps:
(1) A diene compound, a carboxylic acid, an alkaline salt, and a solvent were mixed, and cooled to 10°C;
(2) At a temperature of 20°C and a pH of 3.7, a hydrogen peroxide solution was added thereto dropwise for 4.5 h, and the reaction was continued for another 4 h;
(3) The resulting solution was left for standing to obtain an underlayer of an organic phase I, a washing liquid was added thereto and stirred for 30 min at a pH of 11; and the resulting solution was left for standing to obtain an underlayer of an organic phase II;
(4) The organic phase II was first purified by a vacuum distillation at 42°C for 0.75 h with a vacuum degree of -0.07 MPa, then purified by a one-stage thin film distillation, the temperature of the one-stage thin film distillation was 120°C, the distillation pressure was 85 Pa, and the distillation time was 14 h.

The diene compound was

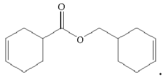

The molar ratio of the carboxylic acid to the diene compound was 2.8:1.

The carboxylic acid was acetic anhydride, and the purity of the acetic anhydride was 98%; and the alkali salt was sodium acetate; and the solvent was toluene.

The concentration of the hydrogen peroxide was 35%; and the molar ratio of the hydrogen peroxide to the diene compound was 4: 1.

The weight ratio of the washing liquid to the solvent was 1:5.

The inorganic alkali solution was a sodium hydroxide solution; and the concentration of the inorganic alkali solution was 30 wt%.

The reducing salt was sodium sulfite; a content of the reducing salt was 1 wt% of the washing liquid.

A cycloaliphatic diepoxide was prepared by the above method.

The cycloaliphatic diepoxide was

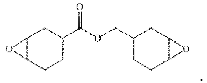

### Example 26

In this example, a method for preparing a cycloaliphatic diepoxide is provided, and its specific implementation is the same as that of Example 1, except that the pressure of the second-stage thin film distillation is 200 Pa.

### Example 27

In this example, a method for preparing a cycloaliphatic diepoxide is provided, and its specific implementation is the same as that of Example 1, except that the organic phase I was first washed with an inorganic alkali solution, and then washed with a reducing salt solution.

### Example 28

In this example, a method for preparing a cycloaliphatic diepoxide is provided, and its specific implementation is the same as that of Example 4, except that the diene compound is 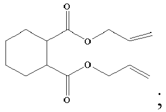 the molar ratio of the carboxylic acid to the diene compound is 1.3:1; the molar ratio of the hydrogen peroxide to the diene compound is 1.7:1; and the cycloaliphatic diepoxide is

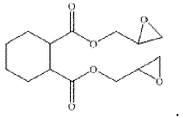

### Example 29

In this example, a method for preparing a cycloaliphatic diepoxide is provided, and its specific implementation is the same as that of Example 4, except that the diene compound is 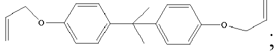 the molar ratio of the carboxylic acid to the diene compound is 2.3:1; the molar ratio of the hydrogen peroxide to the diene compound is 3.4:1; and the cycloaliphatic diepoxide is

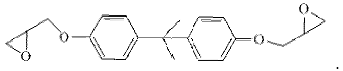

### Example 30

In this example, a method for preparing a cycloaliphatic diepoxide is provided, and its specific implementation is the same as that of Example 4, except that the diene compound is 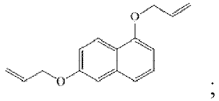 the molar ratio of the carboxylic acid to the diene compound is 2.3:1; the molar ratio of the hydrogen peroxide to the diene compound is 3.7:1; and the cycloaliphatic diepoxide is

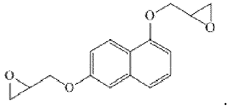

### Example 31

In this example, a method for preparing a cycloaliphatic diepoxide is provided, and its specific implementation is the same as that of Example 29, except that the weight ratio of the washing liquid to the solvent is 1:1.

### Example 32

In this example, a method for preparing a cycloaliphatic diepoxide is provided, and its specific implementation is the same as that of Example 29, except that the reducing salt accounts for 7 wt% of the washing liquid.

### Example 33

In this example, a method for preparing a cycloaliphatic diepoxide is provided, and its specific implementation is the same as that of Example 29, except that the step (4) does not include the two-stage thin film distillation.

### Example 34

In this example, a method for preparing a cycloaliphatic diepoxide is provided, and its specific implementation is the same as that of Example 29, except that the pressure of the second-stage thin film distillation is 200 Pa.

### Example 35

In this example, a method for preparing a cycloaliphatic diepoxide is provided, and its specific implementation is the same as that of Example 29, except that except that the organic phase I was first washed with an inorganic alkali solution, and then washed with a reducing salt solution.

### Performance evaluation

1. Purity test: The purity of the final synthesized cycloaliphatic diepoxides in Examples 1-19 and 21-35 was tested by gas chromatography.
2. Solvent content test: The solvent content of the final synthesized cycloaliphatic diepoxides in Examples 1-19 and 21-27 was tested by gas chromatography; the solvent content of the cycloaliphatic diepoxides after the first-stage thin film distillation in Examples 1-6 was tested by gas chromatography.
3. Yield test: The yields of the final synthesized cycloaliphatic diepoxides in Examples 1-19 and 21-27 were tested according to the mass yield, which refers to the percentage of the mass of the product actually obtained to the mass of the raw materials added to the reactor.
4. Chroma test: The chroma of the final synthesized cycloaliphatic diepoxides in Examples 1-19 and 21-27 was tested according to GB/T22295-2008 Color Measurement Method for Transparent Liquids (Gardner Color).
5. Halogen content test: The total chlorine content in the cycloaliphatic diepoxides synthesized in Examples 28-35 was tested by ion chromatography, and the test results are shown in Table 2.

**Table 1**

| examples | purity (%) | solvent content (%) | | yield (%) | chroma |
|---|---|---|---|---|---|
| | | after the first-stage thin film distillation | after the second-stage thin film distillation | | |
| Example 1 | 98.0 | 2.51 | 0.08 | 106.0 | 0.1 |
| Example 2 | 98.2 | 1.98 | 0.08 | 108.0 | 0.3 |
| Example 3 | 98.1 | 1.85 | 0.08 | 106.0 | 0.4 |
| Example 4 | 97.2 | 2.47 | 0.09 | 102.0 | 0.4 |
| Example 5 | 98.1 | 1.75 | 0.07 | 109.0 | 0.1 |
| Example 6 | 98.2 | 1.92 | 0.08 | 100.0 | 0.1 |
| Example 7 | 99.1 | / | 0.09 | 108.0 | 0.1 |
| Example 8 | 98.8 | / | 0.08 | 101.0 | 0.1 |
| Example 9 | 98.2 | / | 0.08 | 102.0 | 0.2 |
| Example 10 | 98.4 | / | 0.07 | 103.0 | 0.2 |
| Example 11 | 98.5 | / | 0.08 | 102.0 | 0.2 |
| Example 12 | 98.9 | / | 0.07 | 110.0 | 0.1 |
| Example 13 | 98.5 | / | 0.09 | 108.0 | 0.1 |
| Example 14 | 98.7 | / | 0.08 | 109.0 | 0.1 |
| Example 15 | 97.2 | / | 0.18 | 102.0 | 0.3 |
| Example 16 | 97.9 | / | 0.15 | 101.5 | 0.3 |
| Example 17 | 98.1 | / | 0.16 | 101.8 | 0.3 |
| Example 18 | 96.5 | / | 0.23 | 101.6 | 0.3 |
| Example 19 | 97.2 | / | 0.20 | 104.8 | 0.3 |
| Example 21 | 92.0 | / | 0.18 | 94.0 | 0.4 |
| Example 22 | 94.0 | / | 0.17 | 96.0 | 0.3 |
| Example 23 | 87.0 | / | 0.17 | 90.0 | 0.5 |
| Example 24 | 90.0 | / | 0.18 | 98.1 | 0.5 |
| Example 25 | 91.0 | 0.98 | / | 93.2 | 0.5 |
| Example 26 | 97.5 | / | 0.59 | 106.5 | 0.3 |
| Example 27 | 97.0 | / | 0.16 | 104.0 | 0.3 |

Table 1 shows test results of purity, solvent content, yield and chroma of the synthesized cycloaliphatic diepoxides of Examples 1-19 and 21-27. From the test results in Table 1, it can be seen that the cycloaliphatic diepoxides obtained by the methods of the present disclosure have high purity, high yield, low solvent content and low chroma, which are suitable for large-scale industrial production.

**Table 2**

| examples | purity (%) | total chlorine content (ppm) |
|---|---|---|
| Example 28 | 95.5 | 140 |
| Example 29 | 94.0 | 155 |
| Example 30 | 93.8 | 158 |
| Example 31 | 88.9 | 324 |
| Example 32 | 89.2 | 160 |
| Example 33 | 87.0 | 527 |
| Example 34 | 85.9 | 275 |
| Example 35 | 86.8 | 165 |

Table 2 shows the test results of purity and halogen content of the synthesized cycloaliphatic diepoxides in Examples 28-35. From the test results in Table 2, it can be known that the cycloaliphatic diepoxides obtained by the method of the present disclosure have high purity and low halogen content.

The foregoing examples are illustrative only and serve to explain some of the features of the methods described herein. The appended claims are intended to claim the broadest conceivable scope and the embodiments presented herein are merely illustrative of selected implementations.

Accordingly, it is the applicant's intention that the appended claims not be limited by the selection of examples that characterize the disclosure.

## Claims

1. A method for preparing a cycloaliphatic diepoxide comprising the steps of:
- mixing a diene compound, a carboxylic acid anhydride, an alkaline salt, and a solvent, and cooling;
- dropwise adding a hydrogen peroxide solution thereto over 1-12 h, and then continuing the reaction for 3-8 h;
- standing for layering to obtain an underlayer of an organic phase I, washing the organic phase I with a washing liquid, and standing for layering to obtain an underlayer of an organic phase II; and
- purifying the organic phase II;
wherein
the washing liquid includes an inorganic alkali solution and a reducing salt;
the reducing salt accounts for 0.5-5 wt% of the washing liquid; and
in said step of dropwise adding a hydrogen peroxide solution and continuing the reaction, the pH value is kept in a range of 3.0-4.5.

2. The method for preparing a cycloaliphatic diepoxide according to claim 1, wherein a molar ratio of the hydrogen peroxide to the diene compound is (1-5.5): 1.

3. The method for preparing a cycloaliphatic diepoxide according to claim 1, wherein a molar ratio of the carboxylic acid anhydride to the diene compound is (1-3.5): 1.

4. The method for preparing a cycloaliphatic diepoxide according to claim 1, wherein a weight ratio of the washing liquid to the solvent is 1: (2.5-3.5).

5. The method for preparing a cycloaliphatic diepoxide according to claim 1, wherein the purifying includes a vacuum distillation and a two-stage thin film distillation.

6. The method for preparing a cycloaliphatic diepoxide according to claim 5, wherein a temperature of a first-stage thin film distillation is 40-60°C, and a temperature of a second-stage thin film distillation is 80-100°C.

## Patentansprüche

1. Verfahren zur Herstellung eines cycloaliphatischen Diepoxids, umfassend die Schritte:
- Mischen einer Dienverbindung, eines Carbonsäureanhydrids, eines alkalischen Salzes und eines Lösungsmittels und Abkühlen;
- tropfenweise Zugabe einer Wasserstoffperoxidlösung über 1-12 Stunden und anschließendes Fortsetzen der Reaktion für 3-8 Stunden;
- Stehenlassen zur Schichtbildung, um eine Unterschicht aus einer organischen Phase I zu erhalten, Waschen der organischen Phase I mit einer Waschflüssigkeit und Stehenlassen zur Schichtbildung, um eine Unterschicht aus einer organischen Phase II zu erhalten; und
- Reinigen der organischen Phase II;
wobei
die Waschflüssigkeit enthält eine anorganische Alkalilösung und ein reduzierendes Salz;
das reduzierende Salz macht 0,5-5 Gew.-% der Waschflüssigkeit aus; und
in dem Schritt der tropfenweisen Zugabe einer Wasserstoffperoxidlösung und der Fortsetzung der Reaktion wird der pH-Wert in einem Bereich von 3,0-4,5 gehalten.

2. Verfahren zur Herstellung eines cycloaliphatischen Diepoxids nach Anspruch 1, wobei das Molverhältnis des Wasserstoffperoxids zur Dienverbindung (1-5,5): 1 beträgt.

3. Verfahren zur Herstellung eines cycloaliphatischen Diepoxids nach Anspruch 1, wobei das Molverhältnis des Carbonsäureanhydrids zur Dienverbindung (1-3,5): 1 beträgt.

4. Verfahren zur Herstellung eines cycloaliphatischen Diepoxids nach Anspruch 1, wobei das Gewichtsverhältnis der Waschflüssigkeit zum Lösungsmittel 1: (2,5-3,5) beträgt.

5. Verfahren zur Herstellung eines cycloaliphatischen Diepoxids nach Anspruch 1, wobei die Reinigung eine Vakuumdestillation und eine zweistufige Dünnschichtdestillation umfasst.

6. Verfahren zur Herstellung eines cycloaliphatischen Diepoxids nach Anspruch 5, wobei eine Temperatur einer Dünnschichtdestillation der ersten Stufe 40-60°C ist und eine Temperatur einer Dünnschichtdestillation der zweiten Stufe 80-100°C ist.

## Revendications

1. Procédé de préparation d'un diépoxyde cycloaliphatique comprenant les étapes de:
- mélanger un composé diène, un anhydride d'acide carboxylique, un sel alcalin et un solvant, et refroidir;
- y ajouter goutte à goutte une solution de peroxyde d'hydrogène pendant 1 à 12 h, et puis poursuivre la réaction pendant 3 à 8 h;
- laisser reposer pour former des couches pour obtenir une sous-couche d'une phase organique I, laver la phase organique I avec un liquide de lavage, et laisser reposer pour former des couches pour obtenir une sous-couche d'une phase organique II; et
- purifier la phase organique II;
dans lequel
le liquide de lavage comprend une solution alcaline inorganique et un sel réducteur;
le sel réducteur compte pour 0,5 à 5 % en poids du liquide de lavage; et
dans ladite étape consistant à ajouter goutte à goutte une solution de peroxyde d'hydrogène et à poursuivre la réaction, la valeur du pH est maintenue dans une plage de 3,0 à 4,5.

2. Procédé de préparation d'un diépoxyde cycloaliphatique selon la revendication 1, dans lequel le rapport molaire entre le peroxyde d'hydrogène et le composé diène est de (1-5,5):1.

3. Procédé de préparation d'un diépoxyde cycloaliphatique selon la revendication 1, dans lequel le rapport molaire entre l'anhydride d'acide carboxylique et le composé diène est de (1-3,5): 1.

4. Procédé de préparation d'un diépoxyde cycloaliphatique selon la revendication 1, dans lequel le rapport pondéral entre le liquide de lavage et le solvant est de 1 :(2,5-3,5).

5. Procédé de préparation d'un diépoxyde cycloaliphatique selon la revendication 1, dans lequel la purification comprend une distillation sous vide et une distillation en couche mince en deux étapes.

6. Procédé de préparation d'un diépoxyde cycloaliphatique selon la revendication 5, dans lequel une température d'une distillation en couche mince de la première étape est de 40 à 60°C, et une température d'une distillation en couche mince de la deuxième étape est de 80 à 100°C.
